# EUROPEAN PATENT APPLICATION

(11) **EP 1 243 634 A1**
(43) Date of publication of application: **25.09.2002**
(21) Application number: 02252117.3
(22) Date of filing: 25.03.2002
(51) Int. Cl.: C09K 15/08, C07C 39/15, C07C 37/20

(54) **Hindered phenolic antioxidant compositions**

(30) Priority: 23.03.2001 US 278316 P
(71) Applicant: ETHYL CORPORATION, Richmond, Virginia 23219-4304 (US)
(72) Inventor: Gatto, Vincent J., Midlothian, Virginia 23112 (US)
(74) Representative: Cresswell, Thomas Anthony

(57) **Abstract**

Hindered tert-butylphenol antioxidant compositions characterized by a low level of volatile and undesirable single-ring tert-butylphenolic antioxidants and a high level of non-volatile multi-ring (or methylene bridged) tert-butylphenol antioxidants are obtained by reacting a specific mixture of ultra pure ortho-tert-butyiphenol (OTBP), ultra pure 2,6-di-tert-butylphenol (DTBP) and formaldehyde in a solvent in the presence of catalyst. These antioxidants are further characterized by having a low level of 2,6-di-tert-butylphenol, low levels of ortho-tert-butylphenol, and trace levels of 2,4,6-tri-tert-butylphenol.

## Description

### TECHNICAL FIELD

This invention relates to novel hindered phenolic antioxidant compositions containing very low levels of single ring hindered phenolics, processes for making said compositions as well as lubricants and fuels containing said hindered phenolic antioxidant compositions. The hindered phenolic compositions of the present invention provide improved oxidative stability to fuel and lubricant compositions containing said hindered phenolics compared to hindered phenolic compositions containing levels of single ring hindered phenolics outside of the scope of the present invention.

### BACKGROUND

Hindered tert-butylphenolic antioxidants are widely used in the lubricant industry to protect crankcase and industrial oils from oxidation. The proper selection of different antioxidant types is critical if one is to formulate a low cost crankcase or industrial oil. For example, it is well known that the combination of certain hindered phenolic antioxidants with alkylated diphenylamines is highly effective in the stabilization of lubricants. Phenolic antioxidants are also known to function synergistically with zinc dialkyldithiophosphate (ZDDP). Other examples exist where certain hindered phenolic antioxidants are used in combination with a blend of alkylated diphenylamine and phenyl-alpha-naphthylamine to stabilize industrial lubricants. Still other examples exist where a combination of two different hindered phenolic antioxidants are used with alkylated diphenylamines to stabilize crankcase oils against oxidation, e.g., EP 0 456 925 B1.

One of the more widely used phenolic antioxidants is the class of methylene bridged multi-ring phenolic antioxidants having the general structure shown below: 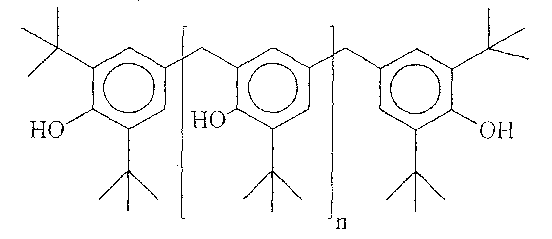 where n equals 0 to 3 or more. As one skilled in the art is well aware, "n" for the individual molecules will be an integer while "n" for the antioxidant composition may be a fraction representing the average value for the composition as a whole. These antioxidants are prepared by reacting a mixture of 2,6-di-tert-butylphenol and ortho-tert-butylphenol with a source of formaldehyde (for example, aqueous formaldehyde or paraformaldehyde), in a reaction solvent and in the presence of an alkylation catalyst. A number of these materials have been made commercially and sold under the tradenames HiTEC® 4702 antioxidant, HiTEC® 4727 antioxidant, HiTEC® 4728 antioxidant, HiTEC® 4738 antioxidant and HiTEC® 4782 antioxidant, all from Ethyl Corporation of Richmond, Virgina. Contaminants in the preparation of these antioxidants include the single-ring phenolics shown below:

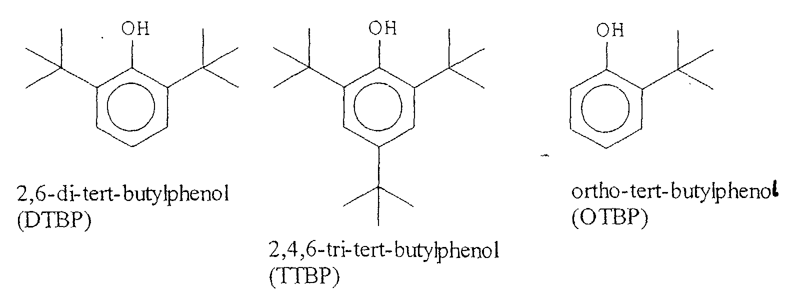

OTBP and DTBP are starting materials for the preparation of the multi-ring hindered phenolic antioxidants that remain in the product after production. TTBP is a material generally found as a contaminant in the OTBP and DTBP used to prepare the multi-ring hindered phenolic antioxidants. These single ring hindered phenolics are soluble in water and are more volatile than the multi-ring hindered phenolic antioxidants. The multi-ring hindered phenolic antioxidants, because of their much higher molecular weight, have a much lower water solubility and are much less volatile.

### SUMMARY OF THE INVENTION

It has been found that a specific mixture of ultra pure ortho-tert-butylphenol (OTBP) and ultra pure 2,6-di-tert-butylphenol (DTBP) when reacted in a solvent in the presence of catalyst produces a mixture containing high levels of desirable multi-ring hindered phenolic antioxidants and low levels of undesirable single-ring hindered phenolic antioxidants. For purposes of the present invention, "ultra pure" refers to OTBP and DTBP monomers containing 0 to 10 ppm tri-tert-butylphenol contamination.

This invention describes a hindered tert-butylphenolic antioxidant composition characterized by a low level of volatile and undesirable single-ring tert-butylphenolic antioxidants and a high level of non-volatile multi-ring (or methylene bridged) tert-butylphenolic antioxidants. In addition, these antioxidants have improved oil solubility and can be easily handled in the liquid form by blending in the appropriate aromatic, paraffinic, or naphthenic process oil. These antioxidants are further characterized by having a low level of 2,6-di-tert-butylphenol, low levels of ortho-tert-butylphenol, and trace levels of 2,4,6-tri-tert-butylphenol. Low levels are defined as less than 3.0 wt % in the neat, i.e. undiluted, antioxidant, while trace levels are defined as less than 50 ppm in the neat, i.e. undiluted, antioxidant.

### DETAILED DESCRIPTION OF THE INVENTION

Hindered phenolic antioxidant compositions containing low levels of 2,6-di-tert-butylphenol (DTBP) and ortho-tert-butylphenol (OTBP), and trace levels of 2,4,6-tri-tert-butylphenol (TTBP) are obtained by reacting a specific mixture of ultra pure DTBP, ultra pure OTBP and a formaldehyde source in a solvent in the presence of a catalyst.

The ultra pure DTBP and the ultra pure OTBP starting materials contain 0 to 10 ppm tri-tert-butylphenol (TTBP).

The ratio of OTBP to total phenols (OTBP / (OTBP + DTBP)) in the starting mixture is in the range of from 0.25 to 0.35 on a weight/weight basis.

A stoichiometric equivalent of formaldehyde or a molar excess of formaldehyde may be used based on the number of active hydrogens in OTBP (2 active hydrogens per phenolic ring) and 2,6-di-tert-butylphenol (1 active hydrogen per phenolic ring). A typical excess ranges from 2 weight % excess to 25 weight % excess. The term "formaldehyde" in the present application includes formaldehyde as well as any source of formaldehyde, for example, aqueous formaldehyde and paraformaldehyde.

The ratio of reaction solvent to total phenols (reaction solvent / (OTBP + DTBP)) is 0.45 or greater. Any polar protic solvent may be used and the reaction solvent can be a mixture of volatile organic solvents and water. Suitable solvents include methanol, ethanol, 1-propanol, 2-propanol, 1-butanol, 2-butanol and t-butyl alcohol. Preferred solvents include ethanol, 1-propanol and 2-propanol.

Any inorganic base may be used as a catalyst. Suitable catalysts include LiOH, NaOH, KOH, CsOH, Mg(OH)₂ and Ca(OH)₂. Preferred catalysts include NaOH and KOH.

In general, the reaction is conducted at or near the reflux temperature of the mixture for from about 30 minutes to 6 hours or more, if necessary. However, reaction temperatures both substantially below and above the reflux temperature will also yield desirable products. Reaction times at these temperatures may be adjusted as is readily understood by one skilled in the art.

Under these reaction conditions an oligomeric product is produced that contains low levels of single ring phenolics, and trace levels of TTBP. The oligomer is composed of phenolic dimers, trimers, tetramers, and high molecular weight phenolics. Characterization of these oligomers by an analytical method known as Low Molecular Weight Gel Permeation Chromatograph (GPC) or Size-Exclusion Chromatography (SEC) is well known in the technical literature [Journal of Chromatography A, 841 (1999) 45-54]. This report also further defines the terms phenolic dimers, trimers, tetramers, and high molecular weight, i.e. high-Mᵣ, and provides the procedure for analyzing samples of multi-ring hindered phenolic antioxidant dimers, trimers, tetramers, and high molecular weight hindered phenolics. High molecular weight hindered phenolics in the publication are defined as any components greater than tetramers, i.e. n > 2.

Under these reaction conditions a multi-ring hindered phenolic reaction product is produced that contains less than 10.0 area % low molecular weight single-ring components having a GPC retention time greater than 22.5 minutes as defined in the method reported above. The product also contains less than 50 ppm TTBP as determined by gas chromatography and mass spectometry (GC-MS). These products are further characterized by having greater than 5 area % and less than 15 area % high molecular weight phenolics (High-Mᵣ) with a retention time less than 20.5 minutes as defined in the method reported above.

The multi-ring hindered phenolic antioxidants produced under these conditions give compositions that are effective antioxidants in lubricants, have low volatility, and low bioaccumulation, and are easily handled as an oil dilution, e.g. 25 to 60 wt. % in an aromatic, naphthenic or paraffinic diluent oil.

These antioxidants have utility in lubricants and fuels. Typical lubricants include passenger car engine oils, heavy duty diesel engine oils, railroad oils, natural gas engine oils, turbine oils, rust and oxidation oils, slideway oils, hydraulic oils, automatic transmission fluids, manual transmission fluids, greases, industrial gear oils, automotive gear oils. Typical fuels include jet fuels, gasolines, heating oils, and diesel fuels.

Important advantages of these products is that they have low volatility and low levels of single-ring hindered phenolics. Lower volatility components are more effective antioxidants because they are not lost due to evaporation during the useful life of the lubricant. They therefore remain in the lubricant to protect the lubricant from the detrimental effects of heat and oxygen, i.e. oxidation.

Although only a few exemplary embodiments of this invention have been described in detail above, those skilled in the art will readily appreciate that many modifications are possible in the exemplary embodiments without materially departing from the novel teachings and advantages of this invention. Accordingly, all such modifications are intended to be included within the scope of this invention as defined in the following claims.

## Claims

1. A hindered phenolic antioxidant composition comprising: 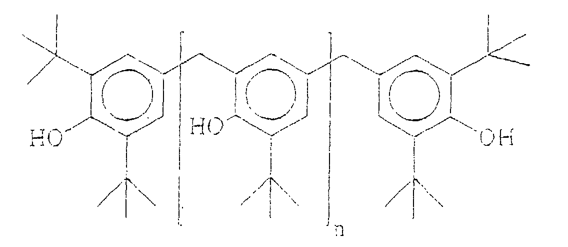 wherein n is 0 to 3 or more; and wherein the composition contains less than 3.0 wt% ortho-tert-butylphenol, less than 3.0 wt% 2,6-di-tert-butylphenol and less than 50 ppm 2,4,6-tri-tert-butylphenol.

2. A process for preparing a hindered phenolic antioxidant composition, which process comprises reacting ortho-tert-butylphenol (OTBP), 2,6-di-tert-butylphenol (DTBP) and a formaldehyde source in a reaction solvent in the presence of a catalyst, wherein:
i) the OTBP and DTBP starting materials each contain from 0 to 10 ppm tri-tert-butylphenol (TTBP);
ii) the ratio of OTBP to total phenols (OTBP/(OTBP+DTBP)) in the starting mixture is from 0.25 to 0.35; and
iii) the ratio of reaction solvent to total phenols (reaction solvent/(OTBP + DTBP)) is 0.45 or greater.

3. A process according to claim 2, wherein the formaldehyde source is selected from formaldehyde, aqueous formaldehyde and paraformaldehyde.

4. A hindered phenolic antioxidant composition obtainable by a process according to claim 2 or 3.

5. A composition according to claim 4 containing less than 3.0 wt% of OTDP, less than 3.0 wt% DTBP and less than 50 ppm 2,4,6-tri-tert-butylphenol.

6. A composition according to any one of claims 1, 4 or 5, which comprises from 25 to 60 wt% of an oil of lubricating viscosity.

7. A composition according to claim 1 or 6, wherein n is from 0 to 4.
